# EUROPEAN PATENT APPLICATION

(11) **EP 2 116 849 A2**
(43) Date of publication of application: **11.11.2009**
(21) Application number: 09159134.7
(22) Date of filing: 30.04.2009
(51) Int. Cl.: G01N 33/487

(54) **Strip supply device**

(30) Priority: 08.05.2008 KR 20080042902
(71) Applicant: Mecasys Co. Ltd., Yuseong-Gu Daejeon-Si 305-325 (KR); Yeongdong Electronics Co., Ltd., Cheoin-Gu Yongin-SI Kyonggi do 449-834 (KR)
(72) Inventor: Jang, Hae Seop, 301-768, Daejeon-Si (KR); Lee, Sang Min, 121-070, Seoul (KR); Kim, Yong Kwan, 306-020, Daejeon-Si (KR); Park, Young Nam, 446-721, Kyunggi-Do (KR); Yun, Yong Chan, 443-470, Kyunggi-Do (KR)
(74) Representative: Barth, Stephan Manuel

(57) **Abstract**

The present invention relates to a strip supply device (100) including: a strip accommodation member (110) having a cassette part (111) having a plurality of rows of vertically laminated strips (10) accommodated side by side therein and a cover part (117) adapted to be slidingly movable to the cassette part (111) so as to open and close the opened portion on the lower side of the cassette part; a take-out module (120) having a plurality of mounting parts (126) each formed to a depth corresponding to the height of one strip (10), so as to take out the strips mounted on the mounting parts (126) by the number of units and to move them to a predetermined position; and a moving module (130) adapted to move the strip accommodation member (110) in one direction so as to place one of the plurality of strip rows correspondingly to the mounting parts (126) of the take-out module (120).

## Description

### BACKGROUND OF THE INVENTION

### Field of the Invention

The present invention relates to a strip supply device, and more particularly, to a strip supply device that is used for an automatic analyzer for a sample liquid such as body fluids and the like, so as to supply a plurality of strips one by one to a predetermined position in a process of testing the sample liquid.

### Background of the Related Art

So as to check the state of a sample liquid such as urine and the like, the sample liquid reacts with a strip, and then, it is checked whether the color of the strip is changed or not. In this manner, however, since one sample liquid should react with one strip, substantially long reaction time is required for a plurality of sample liquids.

In an attempt to solve the above-mentioned problems, thus, an automatic analyzer for sample liquids has been proposed. The automatic analyzer generally includes a strip supply device, a strip-conveying device, a test tube-conveying device, a sample liquid suction/delivery/discharge device, a strip photographer, and a controller.

The strip supply device serves to take out a plurality of strips one by one from a strip accommodation member in which the plurality of strips is accommodated and to supply the strips to a predetermined position.

The strip-conveying device serves to convey the strips supplied from the strip supply device to a position where the strips react with sample liquids and the reaction is detected.

The test tube-conveying device serves to convey a plurality of test tubes in which the sample liquids are stored.

The sample liquid suction/delivery/discharge device serves to suck the sample liquids in the test tubes conveyed from the test tube-conveying device, to deliver the sucked sample liquids to the positions of the pads on the strips conveyed from the strip-conveying device, and to discharge the sample liquids to the pads of the strips.

The strip-photographer serves to photograph the strips so as to detect the reaction of the pads of the strips conveyed from the strip-conveying device.

The controller serves to generally control the operations of each device and function parts in accordance with the embedded operation program and to analyze the photographed information of the strip-photographer.

The automatic analyzer for sample liquids automatically performs the above-mentioned series of processes, such that the time required for the analysis of the sample liquids is more shortened than the conventional manner where the sample liquids directly react with the strips one by one.

The conventional strip supply device used in the automatic analyzer for sample liquids has a structure in which the strips are taken out one by one from the strip accommodation member manufactured in accordance with the automatic analyzer for sample liquids made by manufacturers. That is, each strip is taken at the both sides thereof by means of a gripper-like mechanical device or a pneumatic pressure and is drawn from the strip accommodation member. If the gripper-like mechanical device is used, it is put into the strip accommodation member to take the both sides of the strip, so that relatively large space between the strips should be needed. Therefore, when the gripper-like mechanical device is used, the strip accommodation member becomes complicated in structure, and the structure for activating the gripper-like mechanical device becomes also complicated, thereby causing the manufacturing costs to be more increased. Furthermore, since the gripper-like mechanical device should require relatively large space between the strips, the number of strips to be accommodated in the strip accommodation member is limited, and therefore, the strip accommodation member should be frequently exchanged with new one.

On the other hand, when the strips are taken out by means of the pneumatic pressure, the large space between the strips is not needed when compared with the gripper-like mechanical device, and therefore, a cassette part is simple in structure. However, when the strips are taken out by means of the pneumatic pressure, the parts corresponding to the number of parts activating the gripper-like mechanical device should be provided, so that the structure becomes also complicated. Additionally, when the strips are taken out by means of the pneumatic pressure, a pneumatic pressure part should be separately provided to suck air therefrom, and further, if the pneumatic pressure part is low in stability, a failure rate may be raised.

### SUMMARY OF THE INVENTION

Accordingly, the present invention has been made in view of the above-mentioned problems occurring in the prior art, and it is an object of the present invention to provide a strip supply device having a simple structure that takes out a plurality of strips one by one from a strip accommodation member in which the plurality of strips are accommodated.

To accomplish the above object, according to the present invention, there is provided a strip supply device including: a strip accommodation member having a cassette part having a plurality of vertically laminated strip rows accommodated side by side therein, the cassette part being opened on the lower side thereof, and a cover part adapted to be slidingly movable to the cassette part so as to open and close the opened lower side of the cassette part; a take-out module having a plurality of mounting parts each formed to a depth corresponding to the height of one strip, the mounting parts being adapted to one by one mount the strips dropped by their own weight from the cassette part thereon, so as to take out the strips mounted on the mounting parts by the number of units and to move them to a predetermined position; and a moving module adapted to move the strip accommodation member in one direction so as to place one of the plurality of strip rows correspondingly to the mounting parts of the take-out module.

### BRIEF DESCRIPTION OF THE DRAWINGS

The above and other objects, features and advantages of the present invention will be apparent from the following detailed description of the preferred embodiments of the invention in conjunction with the accompanying drawings, in which:

FIG.1 is a perspective view showing a strip supply device according to the present invention;

FIG.2 is an exploded perspective view showing the interior of the strip supply device in FIG.1;

FIG.3 is a perspective view showing a process of inserting strips into a strip accommodation member in the strip supply device according to the present invention;

FIG.4 is a sectional view taken along the line IV -IV in a state where a cover part of the strip accommodation member of FIG.3 closes an opened portion of a cassette part;

FIG.5 is an exploded perspective view showing a take-out module in the strip supply device of FIG.2;

FIG.6 is a perspective view showing the take-out module of FIG.5;

FIG.7 is a schematic perspective view showing the strip mounted on mounting parts of a roller member of FIG.5;

FIG.8 is a perspective view showing a moving module in the strip supply device of FIG.2;

FIG.9 is a left side view showing the moving module of FIG.8 in a direction of X; and

FIGS.10 to 14 are perspective and sectional views showing the operating processes of the strip supply device according to the present invention, wherein

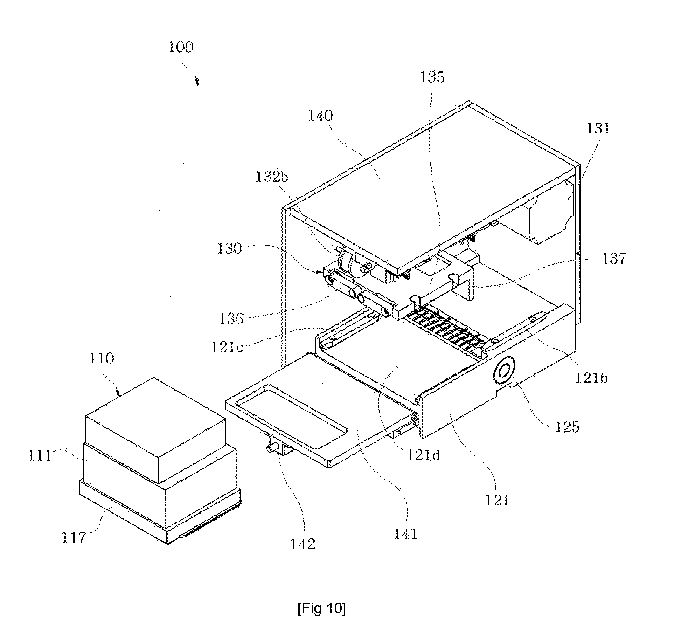

FIG.10 is a perspective view showing a state before the strip accommodation member is coupled to the take-out module in the strip supply device according to the present invention,

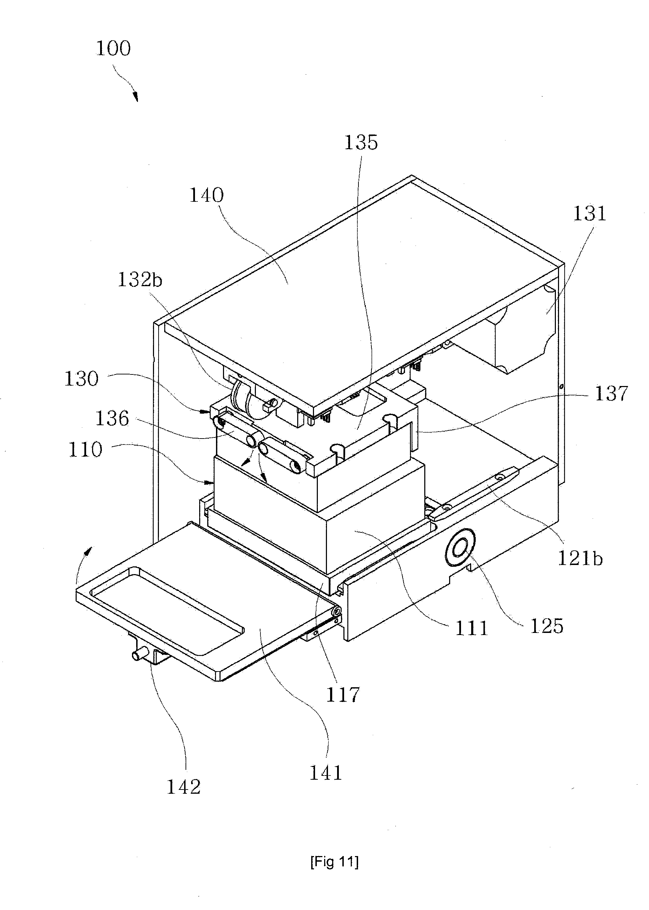

FIG.11 is a perspective view showing a state where the strip accommodation member is coupled to the take-out module in the strip supply device according to the present invention,

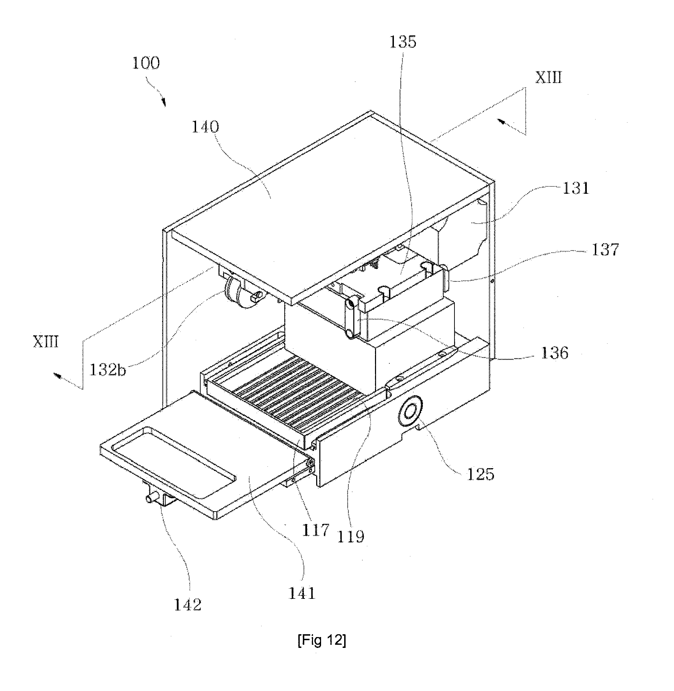

FIG.12 is a perspective view showing a state where the strip accommodation member is moved by means of the moving module in the strip supply device according to the present invention,

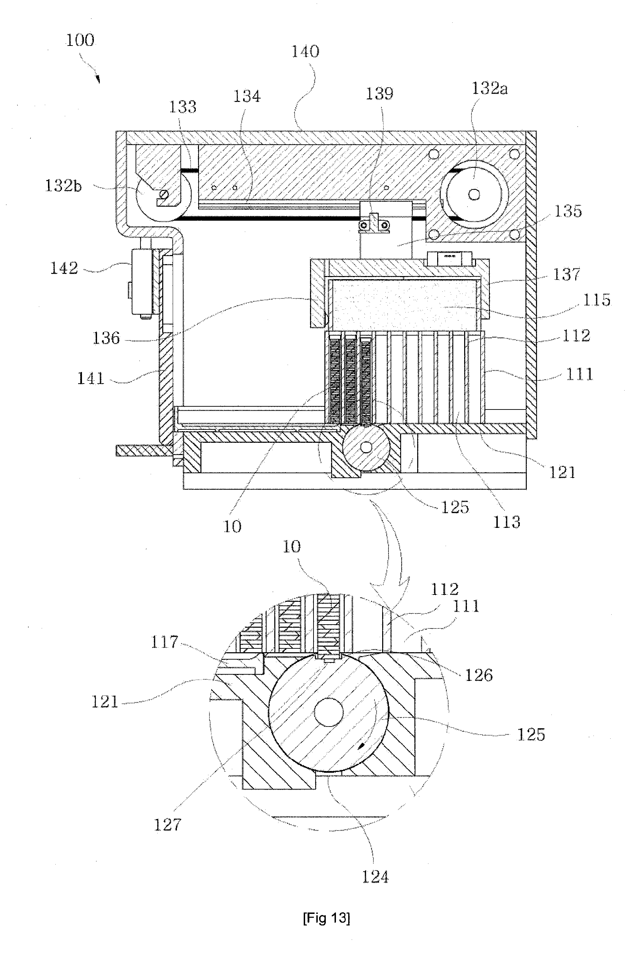

FIG.13 is a sectional view taken along the line XIII -XIII in the strip supply device of FIG.12, and

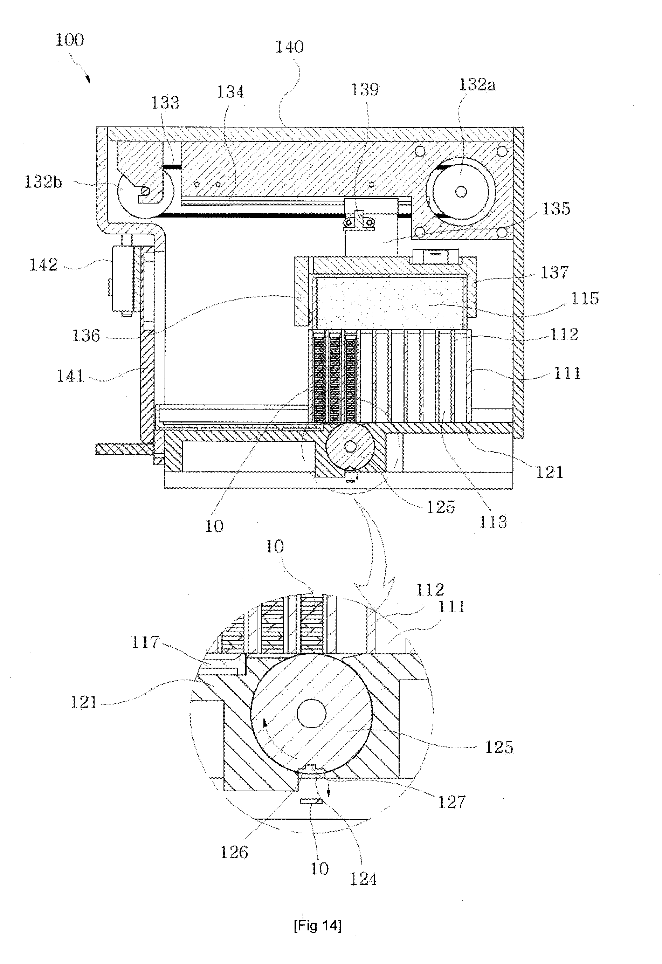

FIG.14 is a sectional view showing one strip is taken out downward by means of the take-out module in the strip supply device of FIG.13.

### DETAILED DESCRIPTION OF THE PREFERRED EMBODIMENT

Hereinafter, an explanation on a strip supply device according to the present invention will be in detail described with reference to the attached drawings.

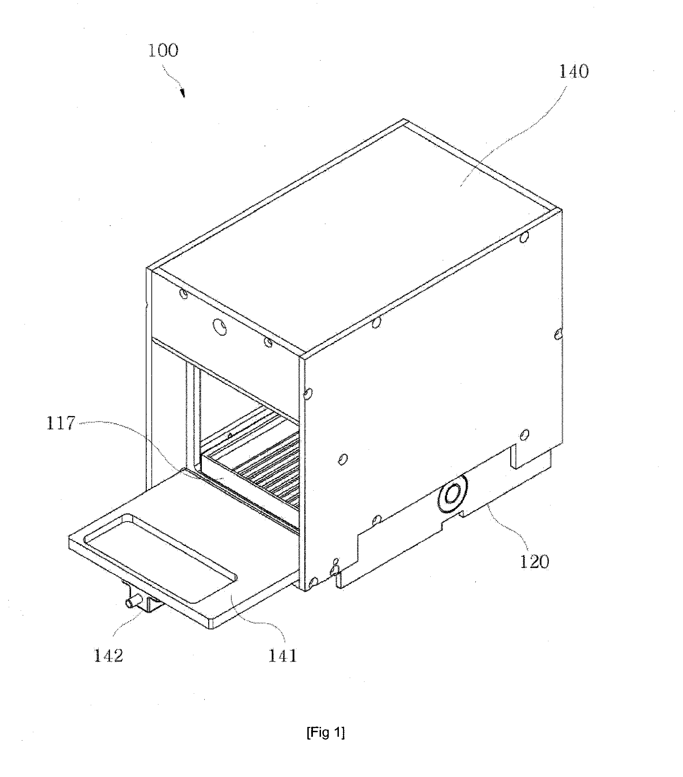

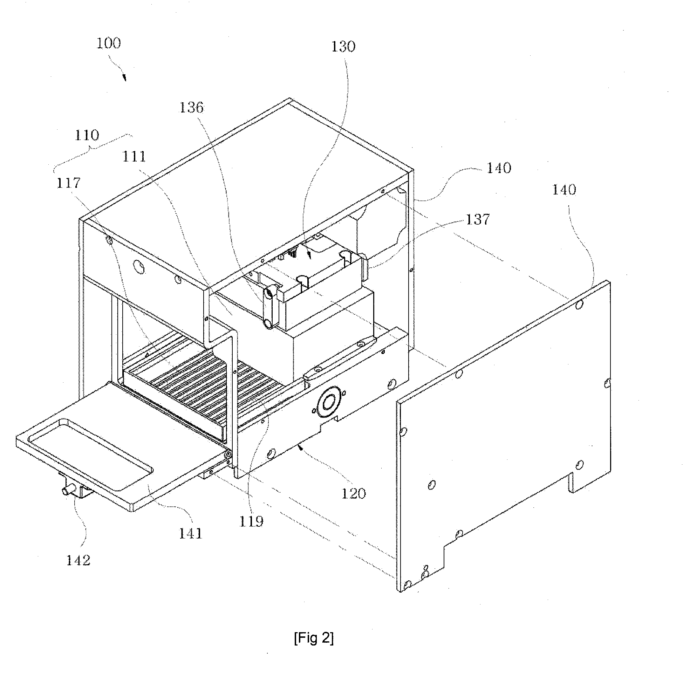

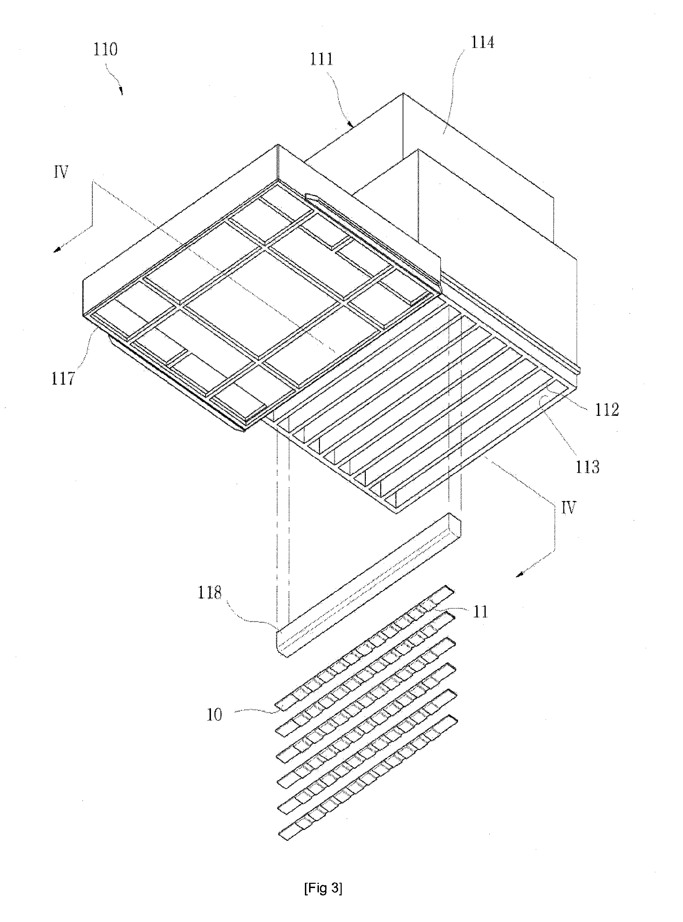

FIG.1 is a perspective view showing a strip supply device according to the present invention, FIG.2 is an exploded perspective view showing the interior of the strip supply device in FIG.1, and FIG.3 is a perspective view showing a process of inserting strips into a strip accommodation member in the strip supply device according to the present invention.

Referring to FIGS.1 to 3, according to the present invention, there is provided a strip supply device 100 including a strip accommodation member 110, a take-out module 120, and a moving module 130.

The strip accommodation member 110 serves to accommodate a plurality of strips 10 therein. The strip accommodation member 110 has a cassette part 111 and a cover part 117.

The cassette part 111 has a plurality of rows of vertically laminated strips 10 accommodated side by side therein, and the cassette part 111 is opened on the lower side thereof. That is, a plurality of partitions 112 are spaced apart from each other by a predetermined distance in the interior of the cassette part 111, and desirably, the plurality of partitions 112 are spaced apart from each other by a little larger distance than the width of each strip 10. The strips 10 are laminated into an accommodation space portion 113 between the partitions 112.

In this case, each strip 10 has a plurality of pads 11 arranged thereon, and biochemical substances having different properties are applied or absorbed on the pads 11. The pads 11 react with a sample liquid such as urine and the like, and then, they are changed or not changed in colors through the reaction with the substances contained in the urine. The cassette part 111 has the plurality of strips 10 laminated thereinto. For example, about 10 accommodation space portions 113 are formed in the cassette part 111, and about 40 strips 10 are laminated in one accommodation space portions 113. Accordingly, since about 400 strips 10 are accommodated in the cassette part 111, there is no need to frequently exchange the strip accommodation member 110 with new one during the test process of the sample liquid.

The cover part 117 is coupled to the cassette part 111 in such a manner as to be slidingly movable thereto, so as to open and close the opened portion of the cassette part 111. According to the present invention, the pads 11 of the strips 10 are directed down into the cassette part 111, and in this case, the cover part 117 has a plurality of contact prevention grooves 119 formed at the inner surface thereof along the sliding direction thereof in such a manner as to be indented on the portions corresponding to the pads 11, thereby preventing the foreign materials of the cover part 117 from coming into contact with the pads 11. In the state where the opened portion of the cassette part 111 is closed by the cover part 117, it is desirable to seal the cassette part 111 from the outside. This is to prevent external air from reacting with the pads 11.

The take-out module 120 serves to take out the strips 10 accommodated in the strip accommodation member 110 one by one by the number of units and to supply the strips 10 to a predetermined position. The take-out module 120 has a plurality of mounting parts 126 formed thereon. Each of the mounting parts 126 is formed to the depth corresponding to the height of one strip 10 so as to one by one mount the strips 10 dropped from the cassette part 111 by their own weight thereon. The take-out module 120 moves the strips 10 mounted on the mounting parts 126 by the number of units to the predetermined position.

The moving module 130 moves the strip accommodation member 110 in one direction and sequentially places the rows of the strips positioned from the front line to the rear line in an advancing direction of the strip accommodation member 110 to the mounting parts 126. Next, the moving module 130 is coupled to the take-out module 120, and alternatively, the take-out module 120 and the moving module 130 are disposed in a separate casing 140.

Referring to the operations of the strip supply device 100 having the above-mentioned structure, the strip accommodation member 110 is coupled to the take-out module 120. Then, if the moving module 130 is coupled to hold one side of the cassette part 111, the moving module 130 moves the cassette part 111 in one direction. In the state where the mounting parts 126 are directed up, the cover part 117 is slidingly moved with respect to the cassette part 111, thereby allowing the opened portion of the cassette part 111 to be opened. Next, the row of strips 10 is dropped by means of its own weight, and one strip 10 is mounted on the mounting parts 126 and is supplied to the predetermined position.

The strip supply device 100 having the above-mentioned structure according to the present invention is simpler in the structure than the conventional strip supply devices, thereby providing substantially low failure rate and manufacturing cost.

Furthermore, the strip supply device 100 according to the present invention provides the strip accommodation member 110 in which a great number of strips 10 are accommodated, thereby preventing the strip accommodation member 10 from being frequently exchanged with new one.

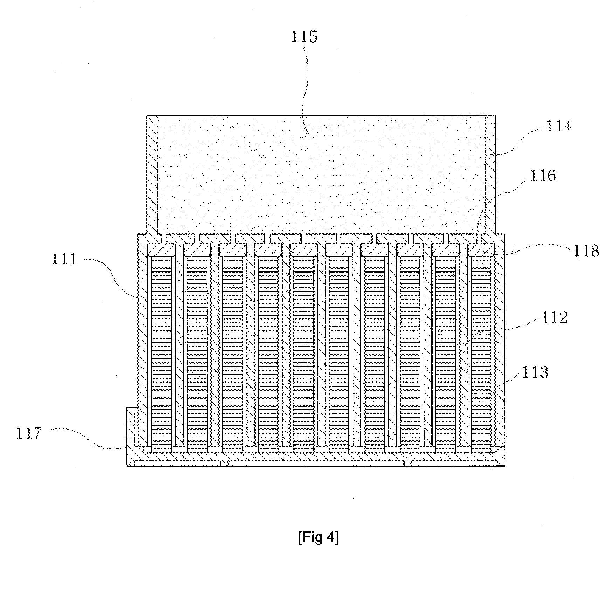

On the other hand, as shown in FIGS.3 and 4, the strip accommodation member 110 has a plurality of weight members 118 provided therein.

Each of the weight members 118 is disposed between the top surface of the inner side of the strip accommodation member 110 and the strip 10.

Each strip 10 is composed of the plurality of pads 11 and a thin sheet on which the pads 11 are attached, for example, an elongated sheet formed of a soft plastic material, so that it is very light in weight. Accordingly, in the state where the strips 10 are accommodated in the inner wall of the cassette part 111, they are dropped one by one from the cassette part 111, and next, one to three strips 10 remain in the cassette part 111. In this case, if the frictional force between the strips 10 and the partitions 112 of the cassette part 111 is higher than the weight of the strips 10, the strips 10 may be not easily dropped from the accommodation space portion 113.

The weight member 118 applies a force in a direction of gravity to the strips 10 by means of its own weight, such that the strips 10 are easily dropped from the accommodation space portion 113. The weight member 118 has a similar size to the strips 10, so as to evenly apply the force caused by its own weight to the undersides of the strips 10.

The height of the weight member 118 is higher than that of the strip 10, and the lower sides of the weight member 118 are rounded. During the operation processes of the strip supply device 100, when the strips 10 in one row of strips out of the strips accommodated in the cassette part 111 are dropped to the take-out module 120, the weight member 118 is mounted on the mounting parts 126 of a roller member 125. Since the height of the weight member 118 is higher than that of the strip 10 and the lower sides of the weight member 118 are rounded, the weight member 118 is not completely housed in the mounting parts 126. Thus, the weight member 118 comes into contact with the top surface of the take-out module 120 and moves the take-out module 120, without any insertion into the interior of the take-out module 120.

On the other hand, as shown in FIG.4, the strip accommodation member 110 further includes a dehumidity part 114.

The dehumidity part 114 is composed of a predetermined housing space portion formed on the top of the strip accommodation member 110. The dehumidity part 114 has at least one dehumidifier 115 disposed at the inside thereof. In this case, desirably, the strip accommodation member 110 has a plurality of dehumidifying holes 116 formed on the top surface thereof. The dehumidifying holes 116 may be formed on the bottom surface between the partitions 112 in the cassette part 111. The dehumidifying holes 116 serve to move the air in the strip accommodation member 110 to the dehumidity part 114. The dehumidifier 115 serves to absorb the water contained in the air existing in the cassette part 111, so as to prevent the water from reacting with the pads 11 of the strip 10.

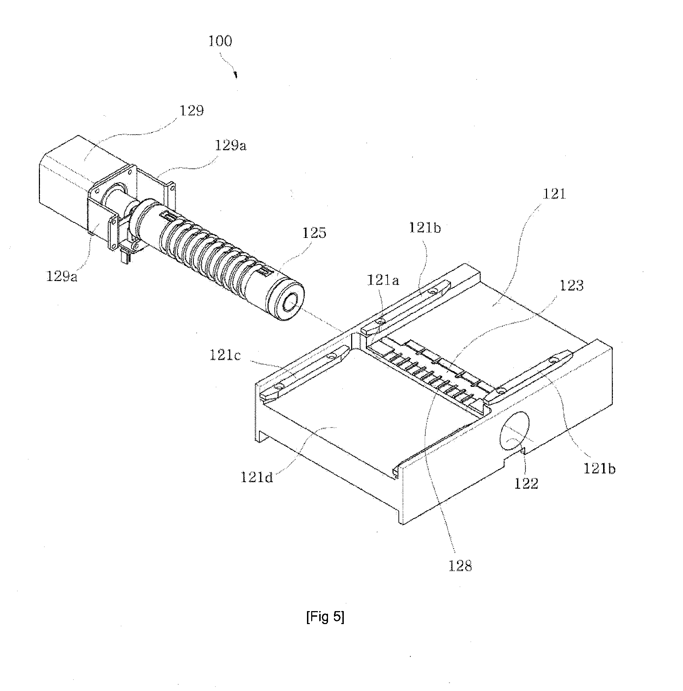

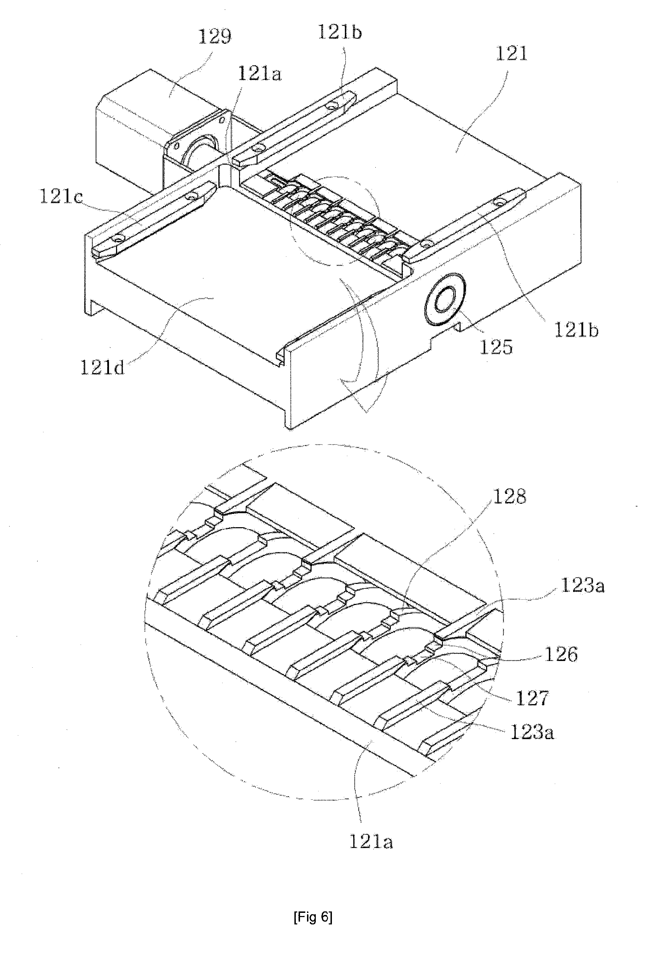

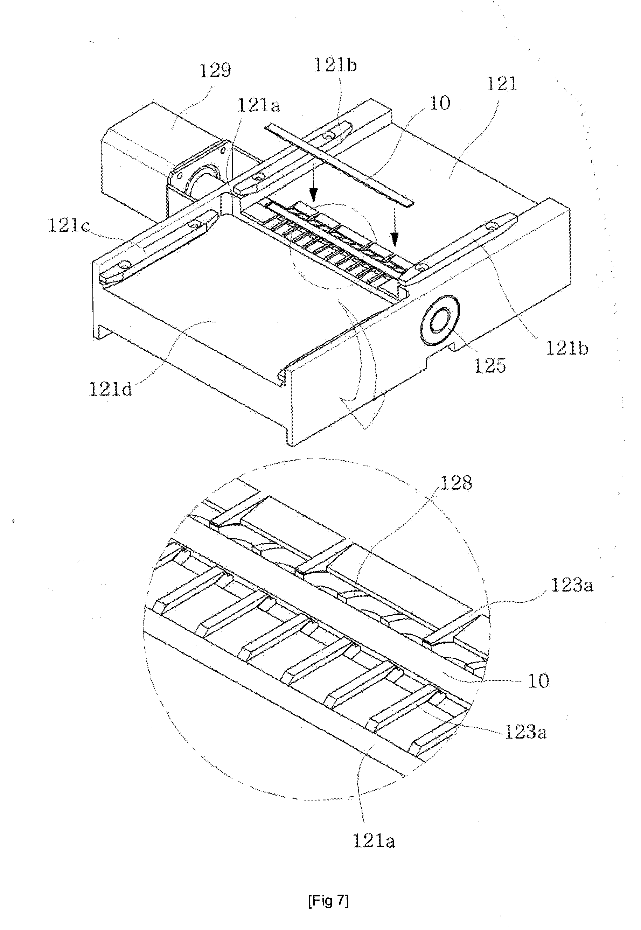

FIG.5 is an exploded perspective view showing a take-out module in the strip supply device of FIG.2, FIG.6 is a perspective view showing the take-out module of FIG.5, and FIG.7 is a schematic perspective view showing the strip mounted on mounting parts of a roller member in FIG.5.

As shown in FIGS.5 to 7, the take-out module 120 includes a roller housing 121, the roller member 125, and an actuator 129.

The roller housing 121 is coupled to the lower side of the strip accommodation member 110, thereby guiding the movement of the cassette part 111. The roller housing 121 has a locking projection 121a and guide parts 121b.

The locking projection 121a serves to restrict the movement of the cover part 117 over a predetermined distance. The locking projection 121a is formed to a portion of the top surface of the roller housing 21 in such a manner as to be adjacent to a coupling face 121d to which the strip accommodation member 110 is coupled. Also, the locking projection 121 is formed to the portion adjacent to the roller member 125 as will be discussed below. Further, the roller housing 121 includes a fixing part 121c mounted at the left and right sides adjacent to the coupling face 121 d. The fixing parts 121c serve to fix the sides of the cover part 117 to allow the cover part 117 to be coupled to the coupling face 121 d.

The guide parts 121b serves to guide the movement of the cassette part 111. The guide parts 121b are formed on the top side of the roller housing 121 in the advancing direction of the cassette part 111. While the cassette part 111 is moved in one direction by means of the moving module 130, the guide parts 121b serve to move the cassette part 111 in the state of being spaced apart from the top side of the roller member 125 by a predetermined distance.

The roller member 125 has the mounting parts 126 each formed to the corresponding depth to the height of one strip 10 so as to one by one mount the strips 10 dropped by their own weight from the cassette part 111 thereon. The roller member 125 is rotatably coupled to the roller housing 121.

The actuator 129 serves to rotate the roller member 125. The actuator 129 has a fixing bracket 129a mounted at one side thereof. The fixing bracket 129a is adapted to fix the actuator 129 to the roller housing 121. If the roller member 125 is rotated, one strip 10 mounted on the mounting parts 126 is moved to a predetermined position, that is, to the lower side according to the present invention.

According to the strip supply device 100 having the above-mentioned structure, the strip accommodation member 110 is always sealed before mounted on the take-out module 120, so that the water contained in the external air does not flow to the inside of the strip accommodation member 110. As soon as the cassette part 111 and the cover part 117 constituting the strip accommodation member 110 are mounted on the take-out module 120 in the state of being coupled to each other, the sample liquid is tested, thereby preventing the strips 10 accommodated in the strip accommodation member 110 from being exposed to the water contained in the external air.

Further, the roller housing 121 includes a receiving part 122, an upper side exposure hole 123, and a lower side exposure hole 124.

The receiving part 122 serves to receive the roller member 125 therein, and thus, the roller member 125 is rotated therein. So as to receive the roller member 125 therein, the receiving part 122 has a somewhat larger diameter than the roller member 125.

The upper side exposure hole 123 has a width corresponding to each of the mounting parts 126, preferably a somewhat larger width than each of the mounting parts 126 in such a manner as to be passed through the top side of the receiving part 122, thereby allowing the mounting parts 126 of the roller member 125 to be exposed to the upper side of the roller housing 121. Thus, the strips 10 dropped from the cassette part 111 are passed through the upper side exposure hole 123 and are mounted on the mounting parts 126. On the other hand, the upper side exposure hole 123 has a plurality of guide protrusions 123a formed therearound. The guide protrusions 123a are formed along the moving direction of the cassette part 111 so as to move the strip 10 therealong, thereby preventing the pads 11 attached on the strip 10 dropped from the cassette part 111 from coming into contact with the top surface of the roller housing 121.

The lower side exposure hole 124 (see FIG. 13) is formed to be passed through the lower side of the receiving part 122, thereby allowing the mounting parts 126 of the roller member 125 to be exposed to the lower side of the roller housing 121. If the strip 10 is mounted on the mounting parts 125 in the state where the mounting parts 126 of the roller member 125 are located at the corresponding position to the upper side exposure hole 123, the actuator 129 is driven to rotate the roller member 125 clockwise by a predetermined angle. For instance, the roller member 125 rotates by 180° according to the present invention, but it is not limited thereto. One strip 10 mounted on the mounting parts 126 escapes from the mounting parts 126 by means of its own weight and is dropped to a predetermined position through the lower side exposure hole 124. Even though not shown in FIGS.5 to 7, the strip 10 dropped through the lower side exposure hole 124 is moved to a sample liquid reaction part in the automatic analyzer for the sample liquid through a separate conveyor and reacts with the sample liquid to be tested.

On the other hand, the above-mentioned roller member 125 further includes a plurality of insertion grooves 127.

The insertion grooves 127 are formed on a portion of the bottom surface of the mounting parts 126, having a smaller width than the strip 10. When the strip 10 is mounted on the mounting parts 126, the insertion grooves 127 serve to reduce the contacting area between the strip 10 and the mounting parts 126, thereby permitting the strip 10 to be easily separated from the mounting parts 126.

On the other hand, the roller member 125 further includes a plurality of isolating protrusions 128 formed therearound.

The isolating protrusions 128 are formed around the roller member 125 in such a manner as to correspondingly come into contact with the face between the adjacent pads 11 attached on the strip 10. The isolating protrusions 128 serve to prevent the roller member 125 from coming into contact with the pads 11 of the strip 10, thereby avoiding the contact between the foreign materials attached around the roller member 125 and the pads 11.

On the other hand, the strip supply device 100 according to the present invention further includes a strip sensor which is not shown in the drawing. The strip sensor detects whether the strip 10 is dropped from the take-out module 120 or not. After the roller member 125 is rotated, if the strip 10 is not dropped from the take-out module 120, the strip sensor sends a signal for moving the cassette part 111 in one direction to the moving module 130. As a result, the moving module 130 moves the cassette part 111 by a predetermined distance to locate one row of strips 10 on the mounting parts 126 of the roller member 125.

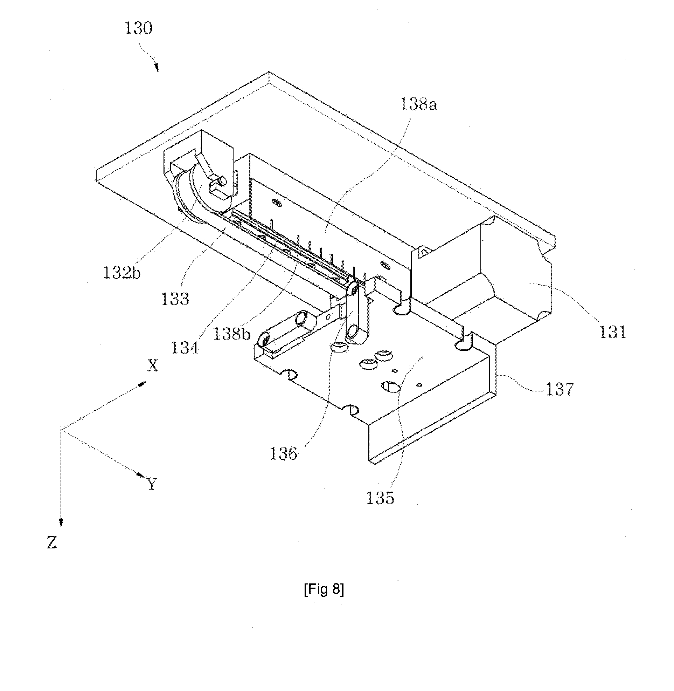

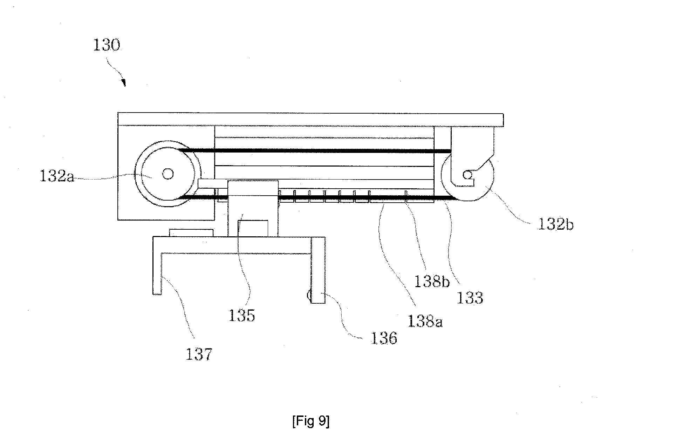

FIG.8 is a perspective view showing a moving module in the strip supply device of FIG.2, and FIG.9 is a left side view showing the moving module of FIG.8.

As shown in FIGS.8 and 9, the moving module 130 includes a driving motor 131, rollers 132a and 132b, a belt 133, an LM guide 134, and an LM block 135.

The roller 132a is coupled to the rotary shaft of the driving motor 131, and another roller 132b is rotatably disposed at a position spaced apart by a predetermined distance from the roller 132a. The rollers 132a and 132b are connected to each other by means of the belt 133, and the LM block 135 is fixedly coupled to a predetermined portion of the belt 133. The LM guide 134 serves to guide the movement of the LM block 135. The LM block 135 has a locking part 137 formed at one side thereof and grip parts 136 formed at the other side thereof. The locking part 137 comes into contact with one side of the cassette part 111, and the grip parts 136 are rotatable to come into contact with the other side of the cassette part 111. Under the above-mentioned structure, the LM block 135 is detachably coupled to the cassette part 111, thereby moving the cassette part 111. The moving module 130 is not limited to the above-mentioned structure, and it may have any structure only if it is detachably coupled to the cassette part 111 to move the cassette part 111 in one direction.

On the other hand, the moving module 130 further includes a position-sensing plate 138a and a position sensor 139 (see FIG.13).

The position-sensing plate 138a is coupled to the LM guide 134 or the driving motor 131 and has a plurality of position-sensing slots 138b spaced apart from each other by a predetermined distance on the lower side thereof.

The position sensor 139 is coupled to the LM block 135 and is moved together with the LM block 135. The position-sensing plate 138a and the position sensor 139 serve to sense whether one row of strips in the cassette part 111 is accurately located at the top side of the mounting parts 126 of the roller member 125.

During the movement of the LM block 135, the position sensor 139 serves to sense the positions of the position-sensing slots 138b of the position-sensing plate 138a. If the position sensor 139 is not accurately located below the position-sensing slots 138b of the position-sensing plate 138a, one row of strips in the cassette part 111 is not in the state of being accurately located at the top side of the mounting parts 126 of the roller member 125. Accordingly, the position sensor 139 sends a motor-driving signal to a controller which is not shown, thereby driving the driving motor 131, and as a result, the position sensor 139 is accurately located below the position-sensing slots 138b of the position-sensing plate 138a.

Moreover, during the movement of the LM block 135, the position-sensing plate 138a and the position sensor 139 serve to prevent one row of strips in the cassette part 111 from escaping from the top side of the mounting parts 126 of the roller member 125 by the sliding between the roller 132a and the belt 133.

On the other hand, the strip supply device 100 according to the present invention further includes an opening/closing cover 141 mounted at one side surface thereof, and the opening/closing cover 141 has a locking part 142 disposed at one side thereof.

FIGS. 10 to 14 are perspective and sectional views showing the operating processes of the strip supply device according to the present invention.

Now, the operating processes of the strip supply device 100 according to the present invention will be in detail explained with reference to the drawings.

As shown in FIG.10, first, the strip accommodation member 110 is coupled to the top side of the take-out module 120 until it is locked to the locking part 137 of the moving module 130. As shown in FIG.11, next, the grip parts 136 of the moving module 130 are rotated to support the rear portion of the cassette part 111, and the opening/closing cover 141 of the strip supply device 100 is closed.

On the other hand, the opening/closing cover 141 is not closed in FIG.12, which is just to show the interior of the strip supply device 100. However, the real operation is performed in the state where the opening/closing cover 141 is closed.

As shown in FIGS. 12 and 13, next, the moving module 130 moves the cassette part 111 such that the strips 10 accommodated in the cassette part 111 are mounted on the mounting parts 125 of the roller member 125.

As shown in FIG. 14, then, the roller member 125 is rotated to discharge one strip 10 to the lower side of the take-out module 120. Even though not shown in FIG.13, the strip 10 dropped through the lower side exposure hole 124 of the roller member 125 is moved to a sample liquid suction/delivery/discharge device in the automatic analyzer for the sample liquid through a separate strip-conveying device and reacts with the sample liquid to be tested.

As described above, the strip supply device according to the present invention has a simpler structure than the conventional ones, thereby providing low failure rate and manufacturing cost.

Additionally, the strip supply device according to the present invention has a multiplicity of strips accommodated therein, thereby having no need to frequently exchange the strip accommodation member.

While the present invention has been described with reference to the particular illustrative embodiments, it is not to be restricted by the embodiments but only by the appended claims. It is to be appreciated that those skilled in the art can change or modify the embodiments without departing from the scope and spirit of the present invention.

## Claims

1. A strip supply device comprising:
a strip accommodation member having a cassette part having a plurality of rows of vertically laminated strips accommodated side by side therein, the cassette part being opened on the lower side thereof, and a cover part adapted to be slidingly movable to the cassette part so as to open and close the opened lower side of the cassette part;
a take-out module having a plurality of mounting parts each formed to a depth corresponding to the height of one strip, the mounting parts being adapted to one by one mount the strips dropped by their own weight from the cassette part thereon, so as to take out the strips mounted on the mounting parts by the number of units and to move them to a predetermined position; and
a moving module adapted to move the strip accommodation member in one direction so as to place one of the plurality of strip rows correspondingly to the mounting parts of the take-out module.

2. The strip supply device according to claim 1, wherein the take-out module comprises:
a roller housing having a locking projection adapted to restrict the movement of the cover part over a predetermined distance and at least one guide part adapted to guide the movement of the cassette part;
a roller member having the mounting parts formed along the length direction thereof, the roller member being rotatably coupled to the roller housing; and
an actuator adapted to rotate the roller member.

3. The strip supply device according to claim 2, wherein the roller housing comprises:
a receiving part adapted to receive the roller member therein, so as to allow the roller member to be rotated therein;
an upper side exposure hole formed to allow the mounting parts of the roller member to be exposed to the upper side of the roller housing, thereby mounting the strips dropped from the cassette part on the mounting parts of the roller member; and
a lower side exposure hole formed to allow the mounting parts of the roller member to be exposed to the lower side of the roller housing, thereby dropping one strip mounted on the mounting parts from the mounting parts when the roller member is rotated in one direction.

4. The strip supply device according to claim 2, wherein the roller member further comprises a plurality of insertion grooves formed on a portion of the bottom surfaces of the mounting parts, having a smaller width than the strip, so as to reduce the contacting area between the strip and the mounting parts.

5. The strip supply device according to claim 1, wherein the strip accommodation member further comprises a plurality of weight members disposed between the top surface of the inner side of the strip accommodation member and the strips, so as to push the strips to a lower side.

6. The strip supply device according to claim 1, wherein the strip accommodation member further comprises: a dehumidity part disposed on the top of the strip accommodation member, the dehumidity part having at least one dehumidifier disposed at the inside thereof; and at least one dehumidifying hole formed on the top surface thereof, so as to move the internal air therein to the dehumidity part.

7. The strip supply device according to claim 1, wherein the cover part comprises a plurality of contact prevention grooves formed at the inner surface thereof along the sliding direction thereof, so as to prevent the contact with the pads of the strip.

8. The strip supply device according to claim 2, wherein the roller member further comprises a plurality of isolating protrusions formed therearound in such a manner as to correspondingly come into contact with the face between the adjacent pads attached on the strip.

9. The strip supply device according to claim 1, further comprising a strip sensor adapted to detect whether the strip is dropped from the take-out module or not.
